# EUROPEAN PATENT APPLICATION

(11) **EP 1 634 887 A2**
(43) Date of publication of application: **15.03.2006**
(21) Application number: 05460018.4
(22) Date of filing: 30.06.2005
(51) Int. Cl.: C07H 15/252

(54) **Process for synthesis of complex 2-deoxy-2-iodo pyranosides of high purity, particularly suitable for manufacturing pharmaceutically pure annamycin**

(30) Priority: 23.07.2004 PL 36925304
(71) Applicant: Zaklad Badawczo-Produkcyjny, 44-121 Gliwice 21 (PL)
(72) Inventor: Szeja, Wieslaw, 44-144 Zernica (PL); Fokt, Izabela, 43-384 Jaworze (PL); Grynkiewicz, Grzegorz, 05-092 Lomianki (PL)
(74) Representative: Maslowski, Andrzej

(57) **Abstract**

The method according to the invention comprises using as glycosyl donors high purity 2-deoxy-2-iodo derivatives of sugars of a defined configuration of C-2 carbon with O-alkyl, S-alkyl, O-aryl, S-aryl, O-heteroaryl, S-heteroaryl, O-acyl, O-silyl, O-N-imido, O-P-(phosphino, phosphono, thiophosphono, selenophosphono) groups at the anomeric position, and subjecting them to a condensation reaction with a compound containing a hydroxyl group or a protected hydroxyl group in the presence of electrophilic reagents, followed by deprotection and isolation of a pharmaceutically pure product. The objective of the present invention is to provide a method for preparing complex glycosides by coupling cyclic multifunctional compounds containing hydroxyl functional groups (glycosyl acceptors, including aglycones of natural origin which are known as constituents of active pharmaceutical ingredients) with diastereoisomerically pure 2-deoxy-2-halopyranosyl derivatives containing anomeric substituent capable of exchange under glycosidating conditions, for example by virtue of activation with electrophilic agents. This method substantially simplifies the synthesis of Annamycin.

The present invention relies on a regio- and stereoselective reaction of cohalogenating 1,2-unsaturated sugars in the presence of hydroxyl group containing compounds, such as: water, alcohols, phenols, carboxylic acids, silanols, phosphinic acids, phosphoric acids, thiophosphoric acids, selenophosphoric acids, followed by separation of the product with proper C-2 carbon configuration required for the synthesis. As the separation of stereoisomers is carried out at the stage of obtaining a relatively inexpensive intermediate in the form of glycosyl donor, the method of the invention substantially reduces the cost of Annamycin manufacture. Other advantages of the synthesis process of the invention consist in the reduction of the number of stages of the antibiotic manufacture process and in yield increase of the final product.

## Description

Subject of this invention is the process for obtaining complex glycosides, by coupling cyclic multifunctional compounds containing hydroxyl functional groups (glycosyl acceptors, including aglycones of natural origin which are known as constituents of active pharmaceutical ingredients) with diastereoisomerically pure 2-deoxy-2-iodopyranosyl derivatives containing anomeric substituent capable of exchange under glycosylating conditions, for example by virtue of activation with electrophilic agents.

Numerous classes of antibiotics and other natural compounds utilized as therapeutic drugs have complex glycosidic structure (an aglycon being polycyclic structural element, such as anthracycline chromophore) [1]. 2-Deoxy-2-halogenated derivatives (glycosides) have not been found in Nature thus far but it has been demonstrated that replacement natural sugar moiety in biologically active compounds by such synthetic construct often exhibit favorable physicochemical and/or pharmacodynamic features. [2-5]

Annamycin (**6**), (+)-4-Demethoxy-7-O-(2,6-dideoksy-2-iodo-α-L-*manno-*pyranosyl) adriamycynone, semisynthetic analog of natural anthracyclines which are widely applied in oncological practice [1], is an example of such favorable structural modification. Annamycin is currenly in clinical trials as a candidate for new antitumor drug.

Synthesis of Annamycin is known from the US Patents [US 4,537, 882; US 5,977,327], which describe procedure of coupling suitably protected anthracycline aglycone (adriamycinone derivative) with 3,4-di-O-acetyl-L-rhamnal, in the presence of N-iodosuccinimide (NIS). In this reaction a mixture of diastereoisomeric 2-deoxy-2-iodopyranosides is formed (theoretically four such isomers are possible), which is difficult to separate and isolation of the desired product (**6**) with α*-manno-* configuration requires special technical operation (column chromatography), which is quite costly. As a result of nonspecific glycosylation step, the final yield of the pure product is rather low.

In the course of study of glycals reactivity, we have found that silanols react with 3,4-di-O-acetyl-L-rhamnal in the presence of NIS, affording in high yield 1-O-silylated 2-iodopyranose with desired configuration L-*manno*. Such intermediate can be considered a glycosylating donor or its immediate precursor. Our invention relies on novel concept, in which instead of forming isomeric products in the glycosylation step, halogenation in position 2 (namely, 2-iodination) precedes coupling with an aglycone. Such sequence allows to use, in reaction with desired complex aglycone, a sugar precursor of very high diastereoisomeric purity (e.g. α*-manno-* in case of Annamycine preparation), which greatly simplifies product isolation procedure.

In fact, in a procedure according to the invention, the diastereomers separation step, which is technically difficult is shifted in synthetic scheme to the point in which only relatively simple and inexpensive intermediates (glycals and silanols) are involved, which prevents consumption of expensive anthracycline aglycone in reaction which is likely to produce coupled side products.
Thus, target glycoside with 2-iodo substituent is obtained according to the invention, starting from a sugar synthon of required configuration (e.g. α*-manno*-, as in case of Annamycin), substituted in the anomeric position with an: O-alkyl, S-alkyl, O-aryl, S-aryl, O-heteroaryl, S-heteroaryl, O-acyl, O-silyl, O-N-imidoyl, O-phosponate, O-phosphate, thiophosphate, selenophosphate, etc., instead of direct NIS promoted condensation of a glycal and an aglycone, in which difficult to separate diastereoisomeric mixture is likely to be formed.

According to the invention, 2-iodinated sugar intermediate with α*-manno-*configuration, with an anomeric substituent selected from the list given above, is obtained in NIS promoted condensation with proper R-OH or R-SH component as the main product, which is isolated and purified by usual means, such as crystallization or chromatography. Such intermediate can further be used as glycosylating agent, either directly, or by transformation into more traditional glycosyl donor, such as glycosyl halide. These procedures are relatively inexpensive to execute, because they do not engage costly anthracycline component.

Convenient intermediates, according to the invention, which are easlily purified to required level of purity by crystallization, are: simple glycosides, anomeric esters, 1-O-sillylated derivatives and thioglycosides, all of them obtainable in reaction of 3,4-di-O-acetyl-L-rhamnal (1), with appropriate thiol or hydroxylic component, in the presence of reagents capable of generating positively charged halogen atom (e.g. iodonium ion), such as N-iodosuccinimide (NIS) or iodocollidinium perchlorate.

In particular, the following O-nucleophilic components have been used, according to the invention, to form easilly crystallizing 2-iodo substituted interemediates from glycals:
- simple and substituted alcohols, aliphatic (e.g. benzyl alcohols) and alicyclic, paricularly these containg an electroacceptor groups, and also water itself;
- phenols and derivatives containing phenolic function as part of a heterocyclic system;
- carboxylic acids, aliphatic, aromatic and heterocyclic;
- silanols, particularly tert-butyldimethysilanol;
- compounds containing N-OH linkage, particularly N-hydroxyimides;
- compounds containing P-OH linkage, particularly derivatives of phosphoric, phosphinic, thiophosphoric or selenophosphoric acids;

In order to obtain proper intermediates which are easy to purify by crystallization and can be activated as glycosyl donors, according to the invention, variety of SH and NH nucleophiles were also applied. Thiols included aliphatic, aromatic (thiophenols) and heterocyclic compounds with SH group, as well as thioloacids. Nitrogen nucleophiles could also be used, subject to selection based on suitable balance of NH nucleophilicity and acidity. Heterocyclic derivatives containing electronoaccepting groups fall into this category.

Enlisted compounds were screened for diastereoselectivity efficiency of glycoside formation and facility of isolation L-*manno-* product from a reaction mixture. Stability under storage condition and susceptibility towards activation under glycosylation conditions used for antracycline aglycones were also taken into account.

Diastereoisomerically pure glycosyl donors with 2-deoxy-2-iodopyranosyl structure, containing anomeric substituents such as: alcoxyl- ; aryloxyl- ; acyloxyl- ; silyloxyl-; phosphate-; etc., are obtained from glycals by addition of chosen O-nucleophile (hydroxyl group containing substrate) in the presence of NIS, followed by appropriate purification procedure (crystallization or chromatography), in yields varying from low (7 - 9%) to good yields (60 - 70%). Application of 1-O silylated derivatives is particularly advantageous. Subject of the invention is best illustrated on the example of Annamycine preparation, as presented on two following schemes, which depict preparation of 1-O-silylated 2-iodo-2-deoxy- intermediate and synthesis of the antibiotic by coupling sugar synthon to anthracycline aglycone.

### Example 1 Synthesis of Annamycin using 1-O-silylated 2-iodopyranose intermediate

### Preparation of 3,4-di-O-acetyl-2-iodo-1-O-tert-butyldimethylsilyl-2,6-dideoxy-α-L-manno-pyranose (2a)

Freshly distilled 3,4-di-O-acetyl-L-rhamnal **1** (4.20 g ; 0.02 M) was disolved in tetrahydrofuran (THF, 28 mL) - acetonitrile (14 mL) mixture, to which *tert-*butyldimethylsilanol (6.2 mL; 0.04 M) was added, followed by N-iodosuccinimide (NIS; 22.5 g ; 0.1 M). The mixture was stirred at room temperature until TLC control (commercial Sio₂ plates developed in hexane - ethyl acetate 4:1) confirmed total consuption of the starting sugar (ca. 8 hrs), after which the reaction flask was put on rotary evaporator and the solvents were removed under reduced pressure. The residue after evaporation was triturated with hexane (100 mL) and the solids were removed by filtration and washed with additional portion of hexane (25 mL). Combined filtrates were washed with 1% aqueous sodium thiosulphate and then water to neutal pH, then dried over anhydrous magnesium suphate and evaporated to dryness. The residue was chromatographed on silica gel column, eluting product with hexane - ethyl acetate 98 : 2 and pooling fractions containing pure compound **2.** Evaporation of the solvent afforded law melting colorless solid, (6.4 g, 68% yield).
¹H-NMR (CDCl₃) δ: 5,41(s, 1H ,H-1), 5,15(dd, 1H, J=9,6 Hz, H-4), 4,65(dd, 1H, J=9,5 Hz, J=4,2 Hz, H-3), 4,47 (dd, J=4,3 Hz, J=1,4 Hz, H-2), 4,07 (dq, 1H, J=9,6 Hz, J= 6,4 Hz, H-5), 2,065, 2,056 ( 2s, 2 x 3H, OAc), 1,20 (d, 3H, J=6,4 Hz, H-6), 0,94 (s, 9H, tBu), 0,13, 0,125 (2s, 2 x 3H, Me₂).

According to the invention it is possible to use anomeric 3,4-di-O-acetyl-2-iodo-1-O-*tert*butyldimethylsilyl-2,6-dideoxy-β-L-*manno*-pyranose or an α, β- mixture in the following coupling reaction leading to Annamycine

### Coupling of 3,4-di-O-acetyl-2-iodo-1-O-tert-butylodimethylosilil-2,6-dideoxy-α-L-manno-pyranose (2a) with protected 4-demethoxy-adriamycinone (3)

Sugar derivative **2a** (1.25g; 2.65 Mmol) and 14-O-(*tert*-butyldiphenyl)silil 4-demethoxy-adriamycynone **3** (0.824g; 1.33 Mmol) were dissolved in anhydrous dichloromethane (20 mL) and molecular sieves 4A (2.0 g) were added. After stirring the mixture for 1 hr at room temperature trimethylsilyl trifluoromethanesulphonate was added, (TMSOTf ; 4.0 Mmol, 0.889 g, 0.8 mL). Progress of the coupling reaction was monitored by TLC (SiO₂ plates developed in hexane - ethyl acetate 1:1). After all aglycone intermediate has been consumed, the reaction mixture was filteres through Cellite pad, which was then washed with dichloromethane (50 mL). Diluted reaction mixture was washed with water until neutral and the organic layer was dried with anhydrous sodium sulphate. After filtering off drying agent and evaporation of the solvent under diminished pressure, residue was purified by column chromatography on silica gel, by eluting with dichloromethane and dichloromethane - acetone Przebieg reakcji kontrolowano metoda TLC. Po 98 : 2. Homogeneous fractions were pooled and evaporated to give 0.935 g of protected annamycine derivative - compound **4** (73% yield), as a red powder.
¹H-NMR(CDCl₃), δ: 13.56, 13.26( 2s, 2 x 1H, 6 ; 11- OH), 8.37÷8.33(m, 2H, H-1.4) 7.87÷7.83(m, 2H, H-2, 3), 7.72÷7.70( m, 4H, H arom. [silyl]), 7.52÷7.38 (m, 6H arom. [silyl]), 5.65 (s, 1H, H-1'), 5.15 (bs, 1H, H-7), 5.11(dd, 1H, J = J = 9.5 Hz, H-4'), 4.93 (d, 1H, J=19.8Hz, 14-CH₂), 4.88 (d, 1H, J=19.8Hz, 14-CH₂), 4.82 (dd, 1H, J = 4.4 Hz, J = 1.4 Hz, H-2'), 4.26 (dd, 1H, J=9.4 Hz, J=4.4 Hz, H-3'), 3.91(dq, 1H, J = 6.3 Hz, J = 9.5 Hz, H-5'), 3.87(s, 1H, 9-OH), 3.06 (d, 1H, J =19.09 Hz, H-10), 2.86 (d, 1H, J = 19.09 Hz, H'-10), 2.10 (d, 1H, J=14.8 Hz, H-8), 2.04 (d, 1H, J =14.8 Hz, H'-8), 2.02, 2.01(2s, 2 x 3H, OAc), 1.14 (s, 9H, t-Bu), 1.12 (d, 3H, J = 6.3 Hz, H-6'),

### Deprotection: removal of C-3'and C-4' acetyl groups

Condensation product **4** (0.78g, 0.81 Mmol) was dissolved in dichloromethane - methanol mixture [CH₂Cl₂/MeOH 2:1 v/v (26 mL)] and sodium methoxide [1N MeONa in MeOH (1.31 Mmol, 1.31 mL)] was added and the reaction mixture was stirred at room temperature until all the substrate (compound **4**) disappeared, as evidenced by TLC (hexane - ethyl acetate 1:1). After that, the reaction mixture was diluted with dichloromethane (50 mL) and neutralized with stoichiometric amount of 1N hydrochloric acid. Obtained solution was poured into water (100 mL), the organic layer was washed twice with water and dried over anhydrous sodium sulphate. Filtering off the drying agent, followed by solvent evaporation afforded annamycine derivative **5** (90 %) which can be purified either by precipitation with hexane, after dissolving in minimal volume of tetrahydrofuran or by column chromatography, using dichloromethane - acetone (98 : 2 followed by 95 : 5 and 9 : 1v/v) or chloroform - methanol mixture (98 : 2 v/v).
¹H-NMR (DMSO-d6) δ: 8.25÷8.24 (m, 2H, H-1,4), 7.96÷7.95 (m, 2H, H-2,3), 7.61÷7.58 (m, 4H, H arom. [silyl]), 7.48÷7.39 (m, 6H, H arom. [silyl]), 5.4 (s, 1H, H-1'), 4.89 (d, 1H, J =19.3 Hz, 14-CH₂), 4.82 (d, 1H, J =19.3Hz, 14-CH₂), 4.26 (d, 1H, J = 3.9 Hz, H-2'), 3.80 (dq, 1H, J = 6Hz, J = 9Hz, H-5'), 3.11(dd, 1H, J = J = 9 Hz, H-4'), 2.91(d, 1H J =18.4Hz, H-10), 2.84 (d, 1H, J =18.4 Hz, H-10), 2.67 (dd, 1H, J = 8.7 Hz, J = 4 Hz, H-3'), 2.00÷1.93 (m, 2H, H-8), 1.00 (s, 9H, t-Bu), 0.97(d, 3H, J = 6.2Hz, H-6').

### Preparation of Annamycin by removal of 14-O-tert-butyldiphenylsilyl ether group

Deacetylation product **5** (0.5 g, 0.57 Mmol) was dissolved in THF (6 mL), then 1N HCl (4.0 mL) was added and the solution was kept at room temperature with TLC monitoring (toluene - acetone 2:1) until complete disappearance of the substrate. Upon completion of de-silylation, the reaction mixture was diluted with chloroform (50 mL) and 10 % aqueous solution of sodium carbonate was added with stirring, until the aqueous layer reached pH 9.0 after which the layers were separated. Aqueous layer was then extracted with chloroform - methanol 95 : 5 v/v,(50 mL) mixture and combined organic extracts were dried over anhydrous sodium sulphate. After filtering off the drying agent and evaporation of the solvents, the residue was chromatographed (Silicagel 60, Merck column), using CHCl₃, CHCl₃/methanol 98:2, and 95:5 as eluents. After pooling homogeneous fraction and evaporation 0.299 g of Annamycin **6**, [(+)-4-Demethoxy-7-O-(2,6-dideoxy-2-iodo-α-L-mannopyranosyl)adriamycynone] was obtained as a red powder ( 82 % yield).
¹H-NMR (DMSO-d6) δ: 8.30 (m, 2H, H-1,4), 8.00 (m, 2H, H-2,3), 5.51 (s, 1H, H-1'), 4.99 (bs, 1H, 1H, H-7), 4.57 (s, 2H, 14-CH₂), 4.33 (d, 1H, J=3.6 Hz, H-2'), 3.97 (m, 1H, H-5'), 3.21 (m, 1H, H-4'), 3.00 (d, 1H J=18.3Hz, H-10), 2.97 (d, 1H, J=18.3Hz, H-10), 2.77 (dd, 1H, J=8.6 Hz, J=3.7 Hz, H-3'), 2.18 (d, 1H, J=12.2 Hz, H-8), 2.13 (dd, 1H, J = 6.1 Hz, J = 12.2 Hz, H-8), 1.22 (d, 3H, J=6.1 Hz, H-6').

### Example 2. Synthesis of Annamycin derivative (4) from anomeric pyranosyl acetate

### Synthesis of 1,3,4-tri-O-acetyl-2,6-dideoxy-2-iodo-α-L-manno-pyranose ( 8 )

Freshly distilled 3,4-di-O-acetyl-L-rhamnal **1** ( 4.2 g; 0.02 Mol) was dissolved with stirring in acetonitrile (40.0 mL) and after cooling the mixture in ice-bath acetic acid ( 10.0 mL; ???Mol) was added, followed by NIS ( 22.5 g; 0.1 mol). Progress of addition was monitored by TLC (SiO₂ plates eluted with hexane - ethyl acetate 1 : 1). Upon reaction completion the mixture was evaporated to dryness under reduced pressure and the residue was triturated with hexane (100 mL), solids were filtered off and washed with more hexane. Combined filtrates were washed with 1% aqueous sodium thiosulphate solution, then with water to neutral pH. After drying and evaporation, the residue was chromatographed on silica gel column, which was eluted with hexane - ethyl acetate 3 : 1. The product was isolated as thick syrup, in 67 % yield.
¹H-NMR ( CDCl₃) δ: 6,34 (d,1H, J=1,0 Hz, H-1), 5,20 (dd,1H, J=9,1 Hz, J=9,6 Hz, H-4), 4,54 (dd, 1H, J=1.2 Hz ,H-2), 4,52 (dd, 1H, J= 4,5 Hz, J=3,4 Hz, H-3), 4,00 (dq, 1H, J= 9,6 Hz, H-5), 2,08, 2,11, 2,15 (3s, 9H OAc).

### Coupling of pyranosyl acetate (8) with adriamycinone derivative (3)

Compound **8** ( 1.21 g, 2.65 Mmol) was dissolved in dichloromethane (20.0 mL), the glycosyl acceptor **3** (0.84 g, 1.33 Mmol), was added, followed by molecular sieves 4A ( 2.0 g) and the mixture was stirred at room temperature for 1 hr, after which time trimethylsilyl triflate was added ( 0.116 ml, 0.6 Mmol) and progress of coupling was monitored by TLC analysis (hexane - ethyl acetate 1: 1). After completion, the reaction mixture was worked up as described in Example 1. Compound **4** (0.78 g; 68% yield) was obtained as red solid, which had identical ¹H NMR spectrum as described above. Stepwise deprotection of compound **4** to obtain Annamycin was performed exactly as described above.

### Example 3 Synthesis of Annamycin derivative (4) from an anomeric phosphate

### Synthesis of 3,4-di-O-acetyl-2,6-dideoxy-2-iodo-β-L-mannopyranosyl-1-O-diphenylphospinate (9)

To freshly distilled L- rhamnal diacetate **1** ( 4.2 g, 0.02 Mol) dissolved in dichloromethane ( 100 mL), diphenylphosphinic acid (21.8 g, 0.1 Mol) was added, followed by dried potassium carbonate (13.8 g, 0.1 Mol) and iodine ( 12.7 g, 0.05 mol).

The reaction mixture was stirred at room temperature for 8 hrs, with monitoring by TLC (hexane - ethyl acetate 1 : 1). After completion of addition, manifested by disappearance of rhamnal, the reaction mixture was washed with aqueous sodium thiosulphate and water until neutral, then dried over sodium sulphate. After filtring off inorganic solid and evaporation of the solvent, product was purified by column chromatography on silicagel bed eluted with hexane - ethyl acetate 3 : 1. Compound **9** was obtained in 70 % yield, in form of amorphous solid.
¹H NMR (CDCl₃) δ : 7,83-7,47( 10H, m, H-aromatyczne), 6,08 (1H, d, J=7,47Hz, H-1), 5,18 (1H, dd, J=9,5 Hz, H-4), 4,68-4,71(2H, m), 4,01 (1H, dd, J=9,9 Hz, J=6,1 Hz, H-5), 2,05, 2,10 ( 2s, 2 x 3H, Me₂), 1,01 ( 3H, d, J=6,1 Hz, H-6).

### Coupling of the phosphinate ester (9) with protected adriamycinone (3)

Anomeric phospinate ester **9** ( 1.48 g, 2.65 Mmol) was dissolved with dichloromethane (20 mL), then aglycone derivative **3** (0.84 g, 1.33 mmol) was added, followed by molecular sieves 4A (2.0 g) and the mixture was stirred at room temperature by 1 hr. Next, trimethylsilyltriflate was added ( 0.135 mL, 0.7 Mmola) and stirring was continued with TLC monitoring. After completion of the reaction ( ca. 2 hrs.) and wor up, according to Example 1, 0.78 g of compound 4 was obtained (68% yield), having the same ¹H NMR characteristic as described above. The two-step deprotection to Annamycin was already described in Example 1

### Example 4. Synthesis of 7-O-(2,6-dideoxy-2-iodo-α-L-mannopyranosyl) adriamycynone

### 1. Coupling of 3,4-di-O-acetyl-2-iodo-1-O-tert-butylodimethylsilyl-2,6-dideoxy-β-L-manno-pyranose (2b) with protected adriamycinone (10)

Sugar synthon **2b** (0.99g, 2.095 Mmola) was dissolved in dichloromethane (20 mL), and 14-O-(tert-butylodiphenyl)silyl-adriamycinone (0.684g, 1.05 Mmol) was added, followed by molecular sieves 4A (1.0 g). After stirring the mixture for 1 hr at room temperature trimethylsilyl trifluoromethanesulphonate was added, (TMSOTf ; 3.12 Mmol, 0.693 g, 0.6 mL). Progress of the coupling reaction was monitored by TLC (SiO₂ plates developed in hexane - ethyl acetate 1:1). After all aglycone intermediate has been consumed, the reaction mixture was filteres through Cellite pad, which was then washed with dichloromethane (50 mL). Diluted reaction mixture was washed with water until neutral and the organic layer was dried with anhydrous sodium sulphate. After filtering off drying agent and evaporation of the solvent under diminished pressure, residue was purified by colunm chromatography on silica gel, by eluting with dichloromethane and dichloromethane - acetone 98:2, and used directly in the next step.

### 2 Deprotection: removal of C-3'and C-4' acetyl groups

**Condensation** product **4** (0.50g, 0.5 Mmol) was dissolved in dichloromethane - methanol mixture [CH₂Cl₂/MeOH 2:1 v/v (15 mL)] and solid potassium carbonate (0.5 g, 3.6 mMol was added and the reaction mixture was stirred at room temperature until all the substrate (compound **4)** disappeared, as evidenced by TLC (hexane - ethyl acetate 1 : 1). After that, the reaction mixture was diluted with dichloromethane (50 mL) and neutralized with stoichiometric amount of 1N hydrochloric acid. Obtained solution was poured into water (100 mL), the organic layer was washed twice with water and dried over anhydrous sodium sulphate. Filtering off the drying agent, followed by solvent evaporation afforded annamycine derivative **12** (0.417 g; 91.8 %) which can be purified either by precipitation with hexane, after dissolving in minimal volume of tetrahydrofuran or by column chromatography, using dichloromethane - acetone (98 : 2 followed by 95 : 5 and 9 : 1v/v) or chloroform - methanol mixture ( 98 : 2 v/v).
¹H-NMR: (CDCl₃) δ: 13.98, 13.41 (2s, 1Hea, 6,11-OH), 7.91 (dd, 1H, J = 7.7 Hz, H-1), 7.78 (dd, 1H, J = 7.7 Hz, J = 8.4 Hz, H-2), 7.70-7.60 (m, 4H, Harom [silyl], 7.48 - 7.37 (m, 7H, H-3 oraz H arom z [silyl], 5.68 (s, 1H, H-1'), 5.13 (dd, 1H, J = 3.9 Hz, J = 2 Hz, H-7), 4.89 (s, 2H, 14-CH₂), 4.48 (dd, 1H, J = 4.1 Hz, J = 1 Hz, H-2'), 4.08 (s, 3H, OMe), 3.91 (s, 1H, 9-OH), 3.74 (dq, 1H, J = 9.2 Hz, J = 6.2 Hz, H-5'), 3.55-3.45 (m, 1H, H-4'), 2.99 (dd, 1H, J =19 Hz, J =1.5 Hz, H-10), 2.80-2.71 (m, 2H, H-10 I H-3'), 2.26 - 2.20 (m, 2H. 3', 4'-OH), 2.09 - 2.00 (m, 2H, H-8), 1.24 (d, 3H, J = 6.2 Hz, H-6'), 1.14 (s, 9H, tBuSi).

### 3.Preparation of Annamycin by removal of 14-O-tert-butyldiphenylsilyl ether group

Deacetylation product **5** (0.5 g, 0.57 Mmol) was dissolved in THF (6 mL), then 1N HCl (4.0 mL) was added and the solution was kept at room temperature with TLC monitoring (toluene - acetone 2:1) until complete disappearance of the substrate. Upon completion of de-silylation, the reaction mixture was diluted with chloroform (50 mL) and 10 % aqueous solution of sodium carbonate was added with stirring, until the aqueous layer reached pH 9.0 after which the layers were separated. Aqueous layer was then extracted with chloroform - methanol 95 : 5 v/v (50 mL) mixture and combined organic extracts were dried over anhydrous sodium sulphate. After filtering off the drying agent and evaporation of the solvents, the residue was chromatographed (Silicagel 60, Merck column), using CHCl₃, CHCl₃/methanol 98:2, and 95:5 as eluents. After pooling homogeneous fraction and evaporation 0.299 g of Annamycin **6,** [(+)-4-Demethoxy-7-O-(2,6-dideoxy-2-iodo-α-L-mannopyranosyl)adriamycynone] was obtained as a red powder ( 82 % yield).

### References

1. F. Arcamone, *Doxorubicin Anticancer Antibiotics,* Academic Press, NY, NY ,1981.
2. W. Priebe (ed.), Anthracycline Antibiotics; New Analogues, Methods of Delivery and Mechanisms of Action, ACS, Washington, DC, 1995; W. Priebe et al., chapter. II, pp. 14-36
3. G. Grynkiewicz, O. Achmatowicz, I. Fokt, W. Priebe, J. Ramza, B. Szechner, W. Szeja, *Wiadomości Chemiczne,* 2002, **56,** 536.
4. D.Horton, W. Priebe, *Carbohydr. Res.,* 136(1985), 391.
5. D.Horton, W.Priebe, O.Varela, *Carbohydr. Res.,* 144(1985) 305.
6. K. Dziewiszek, W. Priebe, USA patent: 5,977,327 (filed 22-07-1997; granted 02-11-1999)
7. D. Horton et al., USA patent: 4,537,882 (filed 08-1985)

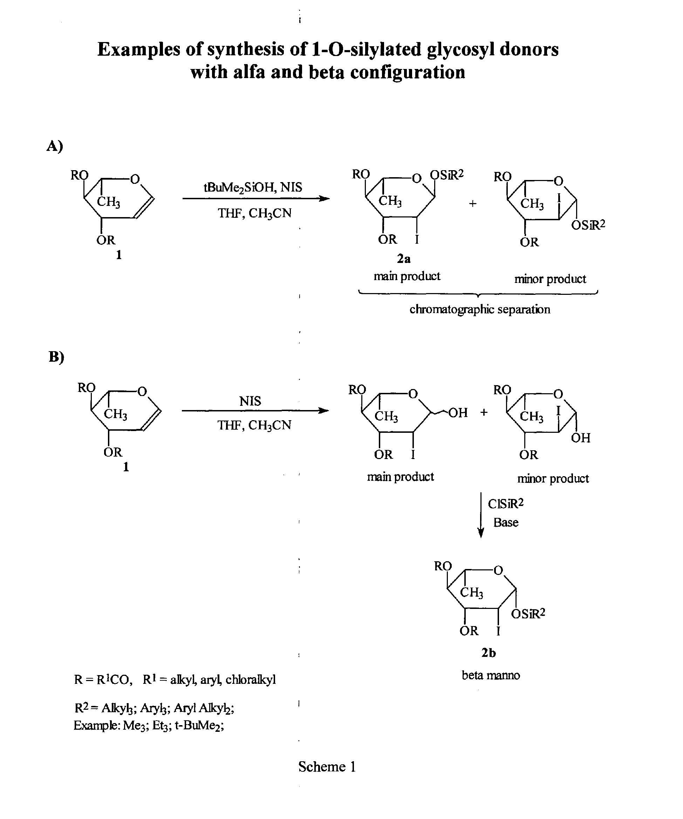

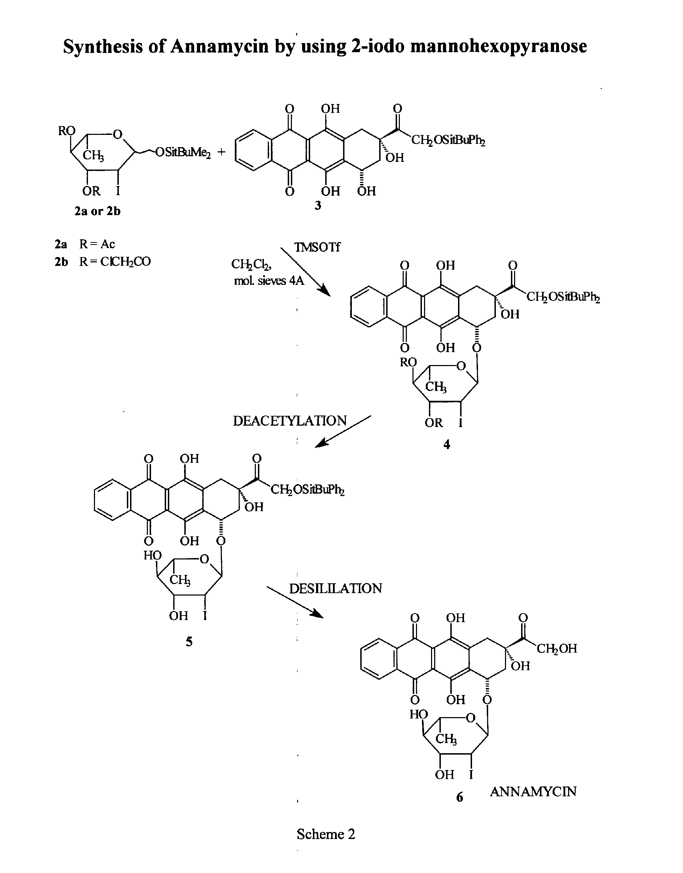

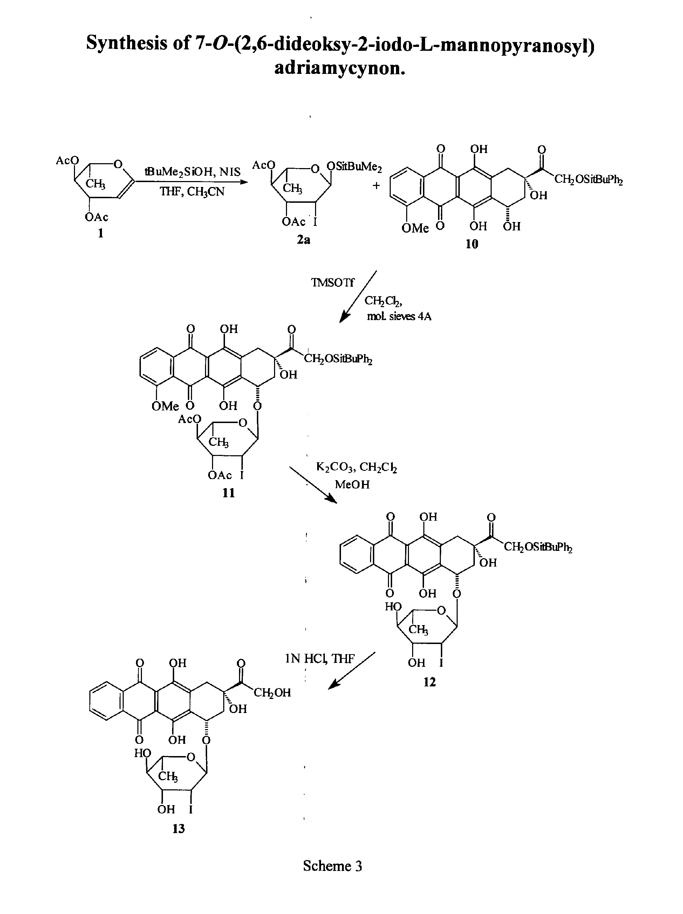

## Claims

1. Process for obtaining diastereomerically pure glycoconjugates, particularly for manufacturing of pharmaceutical purity Annamycin, in a glycosylation reaction of multifunctional organic compounds, in which pure glycosyl donors of defined configuration with 2-deoxy-2-iodo- pyranosyl structure, such as: an anomeric O-alkyl, S-alkyl, O-aryl, S-aryl, O-heteroaryl, S-heteroaryl, O-acyl, O-silyl, O-N-imido, O-P- (phosphino, phosphono, thiophosphono, selenophosphono) are reacted with a component containing unprotected or protected hydroxyl group, in the presence of electrophilic reagents, after which glycosylation the product is deprotected if necessary and isolated in a pure state.

2. Process according to claim 1, in which preferably sugar derivatives containing in the anomeric position substituents: -OSiR'R"₂; -OSiR₃; -OPO(OR)₂; -XPS(XR)₂ ; SR; O-imidates; acyl derivatives (RCO-); or -OPR₂ (in which X = O, S, Se and R = alkyl, aryl, substituted alkyl or aryl, including simple or condensed and substituted heterocyclic rings), are used as glycosyl donors.

3. Process according to claim 1, in which preferable glycosyl donors are 1-O-*tert*butyldimethylsilyl or 1-O-*tert*-butyldiphenylsilyl derivatives of 2,6-dideoxy-3,4-di-O-acetyl-2-iodo-α-L-*manno*- or 2,6-dideoxy-3,4-di-O-acetyl-2-iodo-β- L-*manno-* or their mixtures.
